# EUROPÄISCHE PATENTANMELDUNG

(11) **EP 0 529 470 A1**
(43) Veröffentlichungstag der Anmeldung: **03.03.1993**
(21) Anmeldenummer: 92114034.9
(22) Anmeldetag: 18.08.1992
(51) Int. Cl.: C07D 201/12, C08J 11/16

(54) **Gewinnung von Caprolactam durch thermische Spaltung von Polyamid 6**

(30) Priorität: 30.08.1991 DE 4128788; 07.04.1992 DE 4211609
(71) Anmelder: BAYER AG, 51368 Leverkusen (DE)
(72) Erfinder: Nielinger, Werner, Dr., W-4150 Krefeld (DE); Ostlinning, Edgar, Dr., W-4000 Düsseldorf (DE); Idel, Karsten-Josef Dr., W-4150 Krefeld (DE); Freitag, Dieter, Dr., W-4150 Krefeld (DE); Buysch, Hans-Josef, Dr., W-4150 Krefeld (DE)

(57) **Zusammenfassung**

Gegenstand der Erfindung ist ein Verfahren zur Gewinnung von Caprolactam durch thermische Spaltung von Polyamid in Gegenwart von geringen Mengen an Kaliumcarbonat bei 250 - 320°C sowie ein Verfahren zur Reinigung des entstandenen Caprolactams.

## Beschreibung

Gegenstand der Erfindung ist ein Verfahren zur Gewinnung von Caprolactam durch thermische Spaltung von Polyamid 6 in Gegenwart von geringen Mengen an Kaliumcarbonat bei 250 - 320°C, sowie ein Verfahren zur Reinigung des entstandenen Caprolactams.

Thermoplastische Polyamide werden wegen ihrer guten technologischen Eigenschaften als Rohstoffe für technische Formteile, Fasern und Folien eingesetzt, wobei die Anwendungsbreite der Formteile durch Zusatz von Glasfasern, Füllstoffen oder Schlagzähmodifikatoren noch vergrößert werden kann.

Um die Abfallmengen an Kunststoffen zu reduzieren, wird in zunehmendem Maße auch die Wiederverwendung der Rohstoffe aus gebrauchten Teilen gefordert, und zwar entweder durch Umarbeitung in neue Formkörper oder durch Spaltung in die Monomeren und erneute Polymerisation.

Verfahren zum Abbau von Polyamid 6 zum Caprolactam sind seit langem bekannt. Die Spaltung erfolgt unter der Einwirkung saurer oder basischer Katalysatoren bei erhöhter Temperatur, häufig in Gegenwart von Wasserdampf. In der Ausführung des japanischen Patentes J 50 035-183 werden als Katalysatoren Phosphorsäuren und deren Alkalisalze, Basen wie Natriumhydroxid, Natriumcarbonat und Kaliumhydrogencarbonat, nicht jedoch Kaliumcarbonat genannt. Die Ausbeute an Caprolactam beträgt nach diesem Verfahren jedoch weniger als 50 %.

Die technische Durchführung eines Spaltverfahrens ist in Chem. Ing. Techn. 45, 1510 (1973) beschrieben. Danach wird das Polyamid in einem Reaktionskessel in Gegenwart eines Katalysators mit überhitztem Wasserdampf bei erhöhter Temperatur gespalten. Das entstandene Gemisch Caprolactam - Wasser wird aufkonzentriert und durch ein Oxidationsmittel gereinigt. Schließlich wird das Lactam rektifiziert. Die Oxidation kann mit Wasserstoffperoxid, Natriumhypochlorit oder Kaliumpermanganat in Gegenwart von Calciumoxid erfolgen. (J 52 108-991)

Nach der Europäischen Patentschrift 209 021 kann man Oligomere des Caprolactams in einer Wirbelschicht mit Aluminiumoxid als Katalysator spalten. Das nach diesem Verfahren gewonnene Caprolactam muß selbst nach einer Destillation gemeinsam mit vorgereinigtem Caprolactam aus der Beckmannschen Umlagerung weiter gereinigt werden.

Es wurde nunmehr gefunden, daß man ein Caprolactam in hoher Ausbeute dann erhält, wenn man die Spaltung des Polyamids mit Kaliumcarbonat als Katalysator durchführt und das Caprolactam im Vakuum abdestilliert.

Gegenstand der Erfindung ist also ein Verfahren zur Herstellung von ε-Caprolactam aus Polyamid 6 durch Depolymerisation, indem man das Polyamid unter Zusatz von 0,5 - 2,5 Gewichtsprozent Kaliumcarbonat auf 250 -320°C, vorzugsweise 270 - 300°C, unter Inertgasatmosphäre, vorzugsweise unter N₂, erhitzt und das abgespaltene Caprolactam bei einem verminderten Druck von ≦100 mbar, vorzugsweise von 15 bis 100 mbar, insbesondere von 20 - 90 mbar abdestilliert und das abdestillierte Caprolactam, ggf. unter Zusatz von Additiven, noch einmal fraktioniert.

Durch Verwendung von Kaliumcarbonat anstelle von Natriumcarbonat wird überraschend die Geschwindigkeit der Spaltung von Polyamid 6 und die Ausbeute an Caprolactam beträchtlich erhöht. Dadurch ist es möglich, bei gleichem Durchsatz die Temperatur zu senken, was zu einem farbhelleren und weniger verunreinigten Produkt führt. Dieses kann ohne Oxidation mit Kaliumpermanganat nach der Fraktionierung erneut zur Herstellung hochmolekularer Polyamide benutzt werden. Da eine Zugabe von Wasser oder Wasserdampf beim Spaltprozeß nicht erfolgt, erübrigt sich die Abtrennung des Wassers vor oder während der Fraktionierung des Caprolactams.

Das Verfahren ist auch auf glasfaserhaltiges und füllstoffhaltiges Polyamid 6 anwendbar, ebenso auf Produkte, die Schlagzähmodifikatoren enthalten.

Unter Polyamid 6 wird reines Polyamid 6, wie auch Polyamide verstanden, die zu mehr als 50 Gew.-%, vorzugsweise zu mehr als 80 Gew.-% auf Basis Polyamid 6 offenbart sind.

Bei dem erfindungsgemäßen Verfahren entsteht das ε-Caprolactam in hoher Ausbeute.

Es bedarf jedoch gegebenenfalls noch einer weiteren Reinigung, damit es zu hochmolekularen Polyamiden umgesetzt werden kann. Reinigungsverfahren für Lactame sind bekannt, beispielsweise die Oxidation der wässerigen Lactamlösungen mit Kaliumpermanganat (JA 52108-991), oder eine fraktionierte Destillation über Säuren oder Basen (DE 745 224). Diese Verfahren sind aufwendig und umständlich und liefern zum Teil unbefriedigende Ergebnisse. Beispielsweise wird bei der Polymerisation eines durch thermische Spaltung von unmodifiziertem Polyamid 6 erhaltenen ε-Caprolactams eine relative Viskosität von nur 2,3 erreicht, die nach der Destillation des Lactams über Phosphorsäure auf 3,5 ansteigen kann. Diese Produkte sind nicht zur Herstellung von sehr hochmolekularen Polyamiden geeignet.

Es wurde gefunden, daß man ein ε-Caprolactam erhält, das man auch ohne weitere Reinigungsschritte zu hochmolekularen Polyamiden mit einer relativen Viskosität >3,5 umsetzen kann, wenn man das abgespaltene ε-Caprolactam gleich nach der Entstehung im Vakuum aus dem Reaktionsgemisch abdestilliert, bis das Destillat nicht mehr als 0,2, vorzugsweise nicht mehr als 0,03 mMol basische Anteile in einem Gramm Destillat enthält. Die Bestimmung der basischen Anteile erfolgt durch eine Säure-Base-Titration des Lactam-Destillates mit HCl.

Weiterer Gegenstand der Erfindung ist somit ein Verfahren zur Herstellung von hochreinem ε-Caprolactam aus Polyamid 6, dadurch gekennzeichnet, daß man das aus dem oben beschriebenen Depolymerisationsverfahren erhältliche ε-Caprolactam gleich nach seiner Entstehung bei vermindertem Druck von ≦ 100 mbar vorzugsweise von 15 bis 100 mbar, besonders bevorzugt von 20 bis 90 mbar abdestilliert bis das Destillat nicht mehr als 0,2, vorzugsweise nicht mehr als 0,03 mMol basische Anteile in einem Gramm Destillat enthält.

Auch dieses Ergebnis ist überraschend und nicht vorhersehbar; denn die fraktionierte Destillation eines durch thermische Spaltung gewonnenen ε-Caprolactams führt zu einem Polyamid mit einer relativen Viskosität von nicht mehr als 2,5 und ist für viele praktische Anwendungen daher nicht geeignet.

Die Bestimmung der basischen Anteile erfolgt durch Säure-Base-Titration einer etwa zweiprozentigen, wäßrigen Lösung des ε-Caprolactam-Destillates mit 1 normaler Salzsäure mit einem Mettler-Titrator DL 25.

Weiterer Gegenstand der Erfindung ist die Verwendung des nach dem erfindungsgemäßen Verfahren hergestellten hochreinen ε-Caprolactams zur Herstellung von Polyamiden mit hohem Molekulargewicht und einer relativen Viskosität >3,5, dadurch gekennzeichnet, daß man das aus dem Verfahren erhältliche ε-Caprolactam ohne weitere Reinigungsstufen direkt polymerisiert.

### Beispiel 1

In einem Rundkolben erhitzt man unter Stickstoff 40 g Polyamid 6 mit einer relativen Viskosität von 3,1 (gemessen an einer einprozentigen Lösung in m - Kresol bei 25°C in einem Ubbelohde - Viskosimeter) mit 0,86 g Kaliumcarbonat (2,15 %) auf 320°C (Badtemperatur ). Die Schmelze wird gerührt, nach Erreichen der Badtemperatur wird der Druck schrittweise auf 25 mbar abgesenkt. Bei etwa 60 mbar beginnt das Caprolactam abzudestillieren, die Destillation ist nach etwa 30 min. beendet. Man erhält ein farbloses Destillat in einer Ausbeute von 92 %. Werden 0,43 g Kaliumcarbonat (1,08 %) eingesetzt, sind ca. 55 min für die Destillation erforderlich.

### Beispiel 2

In einem Rührkessel aus Edelstahl wird eine Mischung aus 2,1 kg Polyamid 6 mit einer relativen Viskostät von 3,1 und 36,3 g Kaliumcarbonat mittels eines 320°C heißen Wärmeträgers erhitzt. Nach Erreichen einer Temperatur der Polyamidschmelze von 290°C destilliert man das gebildete Caprolactam im Vakuum ab.

Man erhält in 95 % Ausbeute ein farbloses Destillat mit einer Reinheit von 97 - 98 %, das noch einmal fraktioniert wird.

### Beispiel 3

Man erhitzt 150 g Polyamid 6 mit einer relativen Viskosität von 3,1 (gemessen an einer 1%igen Lösung in m-Kresol bei 25°C in einem Ubbelohde-Viskosimeter) und 2,59 g Kaliumcarbonat auf 300°C (Badtemperatur). 5 Minuten nach Erreichen dieser Temperatur wird unter intensivem Rühren der Schmelze vorsichtig evakuiert. Im Verlaufe von 80 bis 90 Minuten erhält man bei 18 mbar in 93 % Ausbeute ein farbloses Destillat. Das Destillat enthält 0,17 mMol Base pro Gramm Destillat.

Die hydrolytische Polymerisation des Destillates ergibt ein fast farbloses Polyamid mit einer relativen Viskosität von 2,4.

### Beispiel 4

Das Beispiel 4 zeigt die Abhängigkeit der Bildung basischer Produkte von der Zeit. Führt man die Spaltung von Polyamid 6 wie in Beispiel 3 beschrieben, mit der Änderung durch, daß man die Schmelze 1 Stunde lang bei 300°C bis zum Anlegen des Vakuums hält, dann findet man einen Gehalt an basischen Anteilen von 0,24 mMol/g. Die relative Viskosität eines daraus hergestellten Polyamids ist 2,2.

### Beispiel 5

Beispiel 3 wird mit dem Unterschied wiederholt, daß das Destillat vor der hydrolytischen Polymerisation noch einmal fraktioniert destilliert wird. Der Gehalt des Destillats an basischen Anteilen hat nach der Fraktionierung nur unwesentlich abgenommen. Die relative Viskosität des aus dem fraktionierten ε-Caprolactam hergestellten Polyamids beträgt 2,5.

### Beispiele für hochreines ε-Caprolactam

### Beispiel 6

Beispiel 3 wird mit dem Unterschied wiederholt, daß bei der Spaltung die erste Destillatmenge von 40 g abgenommen und ohne weitere Reinigung des ε-Caprolactams, wie in Vergleichsbeispiel 1 beschrieben, hydrolytisch polymerisiert wird.

Mit Salzsäure wurden 0,0086 mMol Base in 1 g Destillat gemessen. Das daraus hergestellte Polyamid ist ein farbloses Produkt mit einer relativen Viskosität von 4,5. Bei der hydrolytischen Polymerisation handelsüblichen ε-Caprolactams wird unter vergleichbaren Bedingungen ebenfalls ein Polyamid mit einer relativen Viskosität von 4,5 erhalten.

### Beispiel 7

Beispiel 6 wird wiederholt, jedoch wird das ε-Caprolactam so lange aufgefangen, bis das Destillat bei der Titration 0,016 mMol Salzsäure pro g Destillat verbraucht.

Nach der hydrolytischen Polymerisation des Lactams erhält man ein farbloses Polyamid mit einer relativen Viskosität von 4,0.

## Patentansprüche

1. Verfahren zur Herstellung von ε-Caprolactam durch Depolymerisation von Polyamid 6 durch alkalisch katalysierte Depolymerisation, dadurch gekennzeichnet, daß man das Polyamid 6 unter einer Inertgasatmosphäre mit 0,5 - 2,5 Gewichtsprozent Kaliumcarbonat auf 250 - 320°C, erhitzt und das abgespaltene ε-Caprolactam bei vermindertem Druck von ≦100 mbar abdestilliert und das Destillat fraktioniert.

2. Verfahren zur Herstellung von Caprolactam gemäß Anspruch 1, dadurch gekennzeichnet, daß glasfaser-und füllstoffhaltiges Polyamid 6 oder schlagzähmodifiziertes Polyamid 6 eingesetzt werden.

3. Verfahren nach Ansprüchen 1 und 2, dadurch gekennzeichnet, daß 0,5 - 2,15 Gew.-% Kaliumcarbonat eingesetzt werden und/oder das Polyamid 6 auf 265 - 300°C erhitzt und/oder das abgespaltene ε-Caprolactam bei 12 bis 100 mbar abdestilliert.

4. Verfahren nach Ansprüchen 1-3, dadurch gekennzeichnet, daß man das Polyamid 6 unter Stickstoffatmosphäre mit 0,5 - 2,0 Gew.-% Kaliumcarbonat auf 265 - 300°C erhitzt und das abgespaltene ε-Caprolactam bei 20 - 80 mbar abdestilliert und das Destillat fraktioniert.

5. Verfahren zur Herstellung von ε-Caprolactam durch Depolymerisation von Polyamid 6 durch Erhitzen des Polyamids mit 0,3 bis 10, vorzugsweise 0,5 bis 2,5 Gewichtsprozent. Kaliumcarbonat unter vermindertem Druck auf 250 bis 300°C, dadurch gekennzeichnet, daß das ε-Caprolactam sofort nach der Entstehung abdestilliert wird, so daß sein Gehalt an Basen nicht mehr als 0,2, vorzugsweise nicht mehr als 0,03 mMol/g Destillat beträgt.

6. Verwendung des nach dem Verfahren gemäß Anspruch 5 hergestellten ε-Caprolactams zur Herstellung von Polyamiden mit hohem Molekulargewicht und einer relativen Viskosität >3,5, dadurch gekennzeichnet, daß man das aus dem Verfahren erhältliche ε-Caprolactam ohne weitere Reinigungsstufen direkt polymerisiert.
